# EUROPEAN PATENT APPLICATION

(11) **EP 4 516 932 A1**
(43) Date of publication of application: **05.03.2025**
(21) Application number: 23796673.4
(22) Date of filing: 13.04.2023
(51) Int. Cl.: C12Q 1/70, G01N 33/569, A61K 39/215

(54) **METHOD FOR CONSTRUCTING HOTSPOT-DERIVED PEPTIDE-NUCLEIC ACID HYBRID MOLECULES ON BASIS OF IN VITRO SELECTION**

(30) Priority: 27.04.2022 KR 20220052389; 05.04.2023 KR 20230044921
(71) Applicant: POSTECH Research and Business Development Foundation, Pohang-si, Gyeongsangbuk-do 37673 (KR)
(72) Inventor: OH, Seung Soo, Pohang-si Gyeongsangbuk-do 37673 (KR); LEE, Min Jong, Pohang-si Gyeongsangbuk-do 37673 (KR)
(74) Representative: Mewburn Ellis LLP
(86) International application number: PCT/KR2023/005036
(87) International publication number: WO 2023/211024

(57) **Abstract**

The present invention relates to a method for preparing an in vitro evolution-basedhotspot-derived peptide-nucleic acid hybrid molecule. According to the method of the present invention, a hotspot-derived peptide-nucleic acid hybrid molecule that can bind with high affinity to viruses and effectively block the binding between the virus and the receptor can be rapidly prepared and screened in response to various virus mutations among numerous candidates. Therefore, it can be used very effectively for the development of therapeutics against various viral mutations.

## Description

### [Technical Field]

The present invention relates to a method for preparing an in vitro evolution-based hotspot-derived peptide-nucleic acid hybrid molecule. The present application claims the benefit and priority to Korean Patent Application No. 10-2022-0052389, filed April 27, 2022, and Korean Patent Application No. 10-2023-0044921, filed April 5, 2023, the disclosure of which is incorporated herein by reference in its entirety.

### [Related Art]

To infect host cells, many viruses specifically recognize cell membrane proteins during their entry into host cells. For example, severe acute respiratory syndrome coronavirus 2 (SARS-CoV-2), as covered by thousands of spike proteins, strongly bind to human angiotensin-converting enzyme 2 (hACE2), the membrane receptor, resulting in membrane fusion. For higher infectivity, viruses including SARS-CoV-2 often mutate, enhancing viral transmissibility and causing immune evasion over time. After first report of SARS-CoV-2 in 2019, many variants have emerged, and variants of concern (VOCs) including alpha (B.1.1.7), beta (B.1.351), gamma (P.1), delta (B.1.617.2) and omicron (B.1.1.529) have independently occurred on different continents. The receptor binding domain (RBD) of the spike protein, which contributes to the specific hACE2 recognition, is known to contain many common mutations. In particular, some mutations (e.g., N501Y) located in the binding hotspot have been identified to increase the binding affinity of the RBD to the target hACE2, thereby increasing the transmissibility of SARS-CoV-2.

The frequent occurrence of variants can be a major obstacle in developing antiviral prevention and treatment strategies. To avoid viral infection, use of affinity reagents can be an effective way by blocking specific interactions between viruses and host cells. For SARS-CoV-2, a number of neutralizing affinity reagents have been developed to recognize various epitopes of the spike protein, some of which are known to partially overlap with the hACE2 contact surface. However, since some escape mutations cause structural changes in the spike protein, it is inevitable that the affinity reagents lose its specific recognition toward their binding site, thereby leading to an reduced neutralization efficacy. As an alternative, non-competitive affinity reagents can be co-administered, and the U.S. Food and Drug Administration (FDA) has issued an emergency use authorization for antibody cocktails such as REGN-COV2 due to the rapid emergence of SARS-CoV-2 variants. As viral variants accumulate escape mutations, they tend to become more resistant to antibody mixtures, while developing stronger binding interaction with the host cell receptor. Therefore, it is necessary to develop effective and efficient neutralizers with high affinity for the target virus, while taking into consideration the binding tolerance to their variants.

### [Detailed Description of the Invention]

### [Technical Problem]

Inspired by the improved receptor recognition of viral variants, the present inventors have invented the generation of receptor-mimetic synthetic reagents that can potently interact with target virus and its variants. Specifically, they focused on peptide motifs on the host cell receptor that contribute significantly to the binding free energy at the center of the virus-receptor interface. Without a stable but insoluble transmembrane domain, the short hotspot peptide cannot maintain optimal binding capacity to the target virus. In this process, it was synergistically integrated with soluble nucleic acids that could act as binding cooperators as well as structural stabilizers. From numerous hotspot peptide-coupled random nucleic acids (~10¹⁴), the hybrid ligands can be readily dicovered by selectively isolating and amplifying aptamer-like scaffolds that maximize the hotspot interaction, which can lead to strong binding to viral variants.

In addition, the inventors successfully created a hACE2 receptor mimetic hybrid ligand that directly interacts with the hotspot of SARS-CoV-2 by using a novel in vitro evolutionary technique called "Hotspot-Oriented Ligand Display" (HOLD). The synergistic interaction between the hotspot peptide and the aptamer scaffold achieved efficient blocking of SARS-CoV-2 by binding to the RBD more effectively compared to reported affinity reagents (e.g., peptides, aptamers or neutralizing antibodies). Furthermore, when recognizing various SARS-CoV-2 variants (e.g., Alpha, Beta, Gamma, Delta and Omicron), the inventors confirmed that the hotspot-binding hACE2 mimic maintained or even enhanced its binding ability toward the SARS-CoV-2 variants, and completed the present invention.

Accordingly, it is an object of the present invention to provide a method of preparing a target protein-binding peptide-nucleic acid hybrid molecule, comprising the following steps:
(a) site-specifically conjugating a hotspot-derived peptide with a random nucleic acid library to prepare a peptide-nucleic acid hybrid;
(b) co-incubating the target protein-coated magnetic beads with the peptide-nucleic acid hybrid of step (a); and
(c) screening peptide-nucleic acid hybrid molecules that bind to target proteins using magnets.

Another object of the present invention is to provide a peptide-nucleic acid hybrid molecule prepared by the method of the present invention.

Another object of the present invention is to provide a composition for neutralizing viruses, comprising a peptide-nucleic acid hybrid molecule prepared by the method of the present invention.

Another object of the present invention is to provide a method of inhibiting or neutralizing viruses, comprising the step of administering a peptide-nucleic acid hybrid molecule prepared by the method of the present invention to an individual in need thereof.

However, the technical challenges of the present invention are not limited to those mentioned above, and other challenges not mentioned is apparent to one having ordinary skill in the art in view of the following description.

### [Technical Solution]

The terms used in this specification are for illustrative purposes only and should not be construed as limiting the invention. Singular expressions include plural ones unless the context clearly indicates otherwise. In this specification, the terms "includes" or "has" and the like indicate the presence of the features, numbers, steps, actions, components, parts or combinations thereof, and do not preclude the possibility of the presence or addition of one or more other features, numbers, steps, actions, components, parts or combinations thereof.

Unless otherwise defined, all terms used herein, including technical or scientific terms, have the same meanings as commonly understood by one of ordinary skill in the art. Terms such as those defined in commonly used dictionaries are to be construed to have meanings consistent with their meanings in the context of the relevant art and are not to be construed in an idealized or overly formal sense unless explicitly defined in this application.

Hereinafter, the detail of the present invention is provided.

The present invention provides a method of preparing a target protein-binding peptide-nucleic acid hybrid molecule, comprising the following steps:
(a) site-specifically conjugating a hotspot-derived peptide with a random nucleic acid library to prepare a peptide-nucleic acid hybrid;
(b) co-incubating the target protein-coated magnetic beads with the peptide-nucleic acid hybrid of step (a); and
(c) screening peptide-nucleic acid hybrid molecules that bind to target proteins using magnets.

In the present invention, the method may be referred to as Hotspot-Oriented Ligand Display (HOLD), which refers to a method of preparing an in vitro evolution-based hotspot-derived peptide-nucleic acid hybrid molecule. The HOLD can ensure that the peptide-nucleic acid hybrid molecule prepared by the method has a strong binding affinity for the RBD due to the synergistic effect between the hotspot peptide and the aptamer scaffold, and especially can ensure that the peptide-nucleic acid hybrid molecule maintains robust binding even when the RBD is mutated.

In other words, the peptide-nucleic acid hybrid molecules produced by the above method are molecules that have been selected, by using numerous nucleic acid libraries which are randomly synthesized, based on in vitro evolutionary techniques, as the ones that can bind most strongly to the RBD site of the virus instead of the receptor.

In the present invention, the manufacturing method may be characterized by further comprising the following steps, but is not limited thereto:
(d) selectively amplifying and cloning the nucleic acid portion of the peptide-nucleic acid hybrid molecule selected in step (c);
(e) preparing the double-stranded DNA generated in step (d) to a single-stranded DNA by using a exonuclease, and purifying it; and
(f) preparing a new randomized nucleic acid library comprising the single-stranded DNA of step (e), and repeating the selection, amplification, and purification processes of steps (a) to (f).

In the present invention, the method may further comprise, after step (a), thermally denaturing and cooling the peptide-nucleic acid hybrid to induce 3D folding of the nucleic acid. This step allows the hotspot peptide to be positioned in the correct location and orientation, and a stable 3D aptamer scaffold may be prepared that can maintain similar or stronger binding properties compared to the receptor.

The selective amplification of the nucleic acid portion of step (d) may be amplified using a forward primer conjugated with one or more functional groups selected from the group consisting of, but is not limited to, hexynyl, 5-octadiynyl and alkyne attached to the 5' end. Because the functional group is bonded to the forward primer, any PCR product that is amplified can be produced with a functional group attached to the 5' end, which allows site-specific conjugation through click reaction, but is not limited thereto.

In the present invention, a hotspot-derived peptide is a peptide residue that is believed to be highly associated with binding between a receptor and its target protein. Since the HOLD method of the present invention is not limited to hotspot-derived peptides, but is universally applicable, the types of hotspot-derived peptides to be used are not limited. Thus, a variety of peptide sites known or to be known in the art to affect binding between a receptor and its target protein may be utilized as hotspot peptides. One preferred embodiment of a hotspot-derived peptide is a hACE2-derived hotspot peptide, or a hACE2-derived RBD-binding peptide, but is not limited thereto.

The hotspot-derived peptide may be an amino acid at a binding site between a target protein and a receptor thereof; all or a portion of an amino acid involved in that binding site in a target protein sequence; or all or a portion of an amino acid involved in that binding site in a receptor sequence.

The hotspot-derived peptide may be a peptide having any functional group that can be used in a click chemistry reaction bonded to the C-terminus or N-terminus of the isolated hotspot-derived peptide, and it may be, for example, a peptide conjugated with at least one functional group selected from the group consisting of azido lysine, azidobutanoic acid, azinoacetic acid, azide, hexynyl, 5-octadiynyl and alkyne, preferably a peptide conjugated (tagged) with an azide at the C-terminus or N-terminus, and more preferably an azide-tagged LGKGDFR (L351 to R357 having SEQ ID NO: 15) peptide, but is not limited thereto.

In the present invention, the target protein may be, but is not limited to, a viral envelope protein, a growth factor protein, a cell membrane receptor, an antibody, an antigenic protein, a protein site for binding a virus to a receptor of a host cell, or a protein containing a receptor-binding domain (RBD) site.

In the present invention, the envelope protein may be selected from the group consisting of, but not limited to, a spike protein, a fiber protein, and an envelope glycoprotein.

In the present invention, the growth factor protein may be epidermal growth factor, fibroblast growth factor, hepatocyte growth factor, nerve growth factor, insulin-like growth factor, vascular endothelial growth factor, platelet-derived growth factor, or bone morphogenetic protein.

In the present invention, the cell membrane receptor can be any receptor to which viral protein and growth factor protein binds, and can be independent receptor present on the cell membrane, such as, PD-1 and PD-L1, but not limited thereto.

In one embodiment of the present invention, a hotspot peptide-nucleic acid hybrid molecule can be prepared for the development of coronavirus therapeutics, wherein the hotspot-derived peptide is seven amino acid fragments of L351 to R357 (LGKGDFR, SEQ ID No. 15) of the RBD contact residues of the receptor hACE2. The target protein may be, a spike protein, preferably, but is not limited to, a receptor-binding domain (RBD) of a spike protein. Thus, the prepared hotspot peptide-nucleic acid hybrid molecule may inhibit the interaction between the RBD of SARS-CoV-2 and the hACE2 receptor.

In the present invention, an aptamer scaffold is a nucleic acid that is linked to a peptide, assists in the function of that peptide, and is capable of binding strongly and specifically to a particular molecule. In one embodiment of the present invention, also referred to as a peptide-based aptamer scaffold, it is combined with a hotspot-derived peptide to have viral neutralization function, and a hybrid molecule of the peptide and the aptamer scaffold can bind to a target protein.

In the present invention, the randomized nucleic acid library may be a nucleic acid incorporating any functional group that can be used in a click chemistry reaction at the 5' end, for example, a nucleic acid including a single-stranded nucleic acid with one or more functional groups selected from the group consisting of, but not limited to, hexynyl, 5-octadiynyl, alkyne, azido lysine, azidobutanoic acid, azinoacetic acid, and azide, preferably, a nucleic acid to which a hexynyl group incorporated at the 5' end. Furthermore, the functional groups incorporated to the hotspot-derived peptide and the nucleic acid library may be interchangeable. Moreover, the random nucleic acid library may be characterized as comprising random nucleic acids having a structure as shown below:
5'-functional group-forward primer-[Nₓ]-reverse primer-3',
wherein the functional group is selected from the group consisting of hexynyl, 5-octadiynyl, alkyne, azido lysine, azidobutanoic acid, azinoacetic acid, and azide,
wherein N is A, T, C or G, and x is an integer of 25 to 100.

The length of the random nucleic acid library is minimized for precise binding to a target protein having a diameter of 10 nm or less, and may be any length suitable for maximizing the diversity of the library, but specifically, the number of sequences in the random nucleic acid library may be, but is not limited to, 55 to 130, 55 to 110, 55 to 90, 55 to 80, 60 to 130, 60 to 110, 60 to 90, 60 to 80, 65 to 130, 65 to 110, 65 to 90, 65 to 80, 65 to 75, or 70.

Further, the x may be, but is not limited to, 25 to 100, 25 to 80, 25 to 60, 25 to 50, 30 to 100, 30 to 80, 30 to 60, 30 to 50, 35 to 100, 35 to 80, 35 to 60, 35 to 50, 35 to 45, or 40.

A preferred embodiment of the present invention utilizes a forward primer represented by SEQ ID No. 1 and a reverse primer represented by SEQ ID No. 2, wherein the randomized nucleic acid library includes randomized nucleic acids of the structural formula GGAAGAGATGGCGAC-N₄₀-AGCTGATCCTGATGG.

In the present invention, the hotspot-derived peptide and the single-stranded nucleic acid may be characterized by, but are not limited to, being site-specifically coupled by a click reaction. The click reaction may be, but is not limited to, a reaction in which the functional group of the hotspot-derived peptide and the functional group of the nucleic acid are chemically crosslinked to each other or bonded by copper-catalyzed cycloaddition in a short time.

In the present invention, the target protein may be, but is not limited to, a viral envelope protein, a growth factor protein, a cell membrane receptor, an antibody, or an antigenic protein. The viral envelope protein may be selected from the group consisting of, but not limited to, a spike protein, a fiber protein, and an envelope glycoprotein.

In the present invention, the viruse may be selected from the group consisting of, but is not limited to, Coronavirus, influenza virus, Hepatitis virus, Human Immunodeficiency virus (HIV), Human Papillomavirus, Herpesvirus, Ebolavirus, MERS virus, Rotavirus, Hantavirus, Monkeypox virus, Adenovirus, Rabies virus, and Norovirus.

In the present invention, the coronavirus may be one selected from the group consisting of, but not limited to, human coronavirus 229E (HCoV-229E), human coronavirus OC43 (HCoV-OC43), severe acute respiratory syndrome coronavirus (SARS-CoV), human coronavirus NL63 (HCoV-NL63, New Haven coronavirus), human coronavirus HKU1, Middle East respiratory syndrome coronavirus (MERS-CoV), severe acute respiratory syndrome coronavirus 2 (SARS-CoV-2), and variants thereof, wherein the SARS-CoV-2 variant may be any one selected from the group consisting of, but not limited to, alpha (α), beta (β), gamma (γ), delta (δ), and omicron (o) variants.

In the present invention, a "coronavirus" is the genus of the virus species included in the Nidovirales order, Coronaviridae family, Coronavirinae or Torovirinae subfamily. Coronavirus is a virus enveloped by +ssRNA and a helically symmetric nucleopeptide. In addition, severe acute respiratory syndrome coronavirus 2 (SARS-CoV-2) is the seventh coronavirus to infect humans so far, and the others are human coronavirus 229E (HCoV-229E), human coronavirus OC43 (HCoV-OC43), severe acute respiratory syndrome coronavirus (SARS-CoV), human coronavirus NL63 (HCoV-NL63, New Haven coronavirus), human coronavirus HKU1, and Middle East respiratory syndrome coronavirus (MERS-CoV). The proteins that contribute to the overall structure of coronaviruses are the spike, envelope and nucleocapsid. In the case of SARS coronavirus, an established ligand receptor domain on the spike (S) mediates the attachment of the virus with its cellular receptor, angiotensin converting enzyme 2 (ACE2). Some coronaviruses (especially beta-coronavirus subgroup) also have short spikes of a protein called antisense esterase. Coronaviruse can cause viral pneumonia or secondary bacterial pneumonia, and they can also cause direct viral bronchitis or secondary bacterial bronchitis. The human coronavirus discovered in 2003 is the severe acute respiratory syndrome coronavirus (SARS-CoV), which causes severe acute respiratory syndrome (SARS), an infection of the upper and lower respiratory tract.

Also, as used herein, the term "SARS-CoV-2" refers to a new coronavirus, which is an RNA virus that is a variant of SARS and MERS. SARS-CoV-2 shares about 79.7% sequence identity with SARS and about 50% with MERS. However, in contrast to SARS and MERS, the spike glycoprotein of 2019-nCoV forms a structure with one RBD domain that protrudes upward, resulting in 100 to 1,000 times stronger binding to its target receptor, ACE2 (angiotensin). This stronger binding allows for greater penetration into the cell, leading to increased infectivity.

Furthermore, for the purposes of the present invention, variants of severe acute respiratory syndrome coronavirus 2 (SARS-CoV-2) indicates variants of COVID-19. The variants are divided into major variants [Variant of Concern (VOC)] and other variants [Variant of Interest (VOI)], and the variants are named with the Greek alphabet (alpha, beta, gamma, etc.) to prevent the use of localized names and to facilitate communication. The major variants are those that have been identified as having increased transmission or negative epidemiologic changes, increased pathogenicity or clinically significant changes in disease severity, or decreased effectiveness in diagnostics, vaccines, therapeutics, etc. Currently, VOCs are omicron variants, and in the past, they have included alpha, beta, gamma, and delta variants. Specifically, the alpha (α) variant of SARS-CoV-2 was discovered in the United Kingdom in September 2020 and has a phylogenetic classification of B.1.1.7. In addition, the beta (β) variant of SARS-CoV-2 was identified in South Africa in May 2020 and has a phylogenetic classification of B.1.351. Furthermore, the gamma (γ) variant of SARS-CoV-2 was identified in Brazil in November 2020 and has a phylogenetic classification of P.1. Furthermore, the delta (δ) variant of SARS-CoV-2 was identified in India in October 2020 and has a phylogenetic classification of B.1.617.2. In addition, the omicron (o) variant of SARS-CoV-2 was identified in multiple countries in November 2021 and has a phylogenetic classification of B.1.1.529.

In the present invention, the peptide-nucleic acid hybrid molecule may satisfy any of the following features, but is not limited to: (a) nuclease resistance; and (b) serum stability. In addition, it can neutralize viruses and provide electrostatic interactions between RBDs of target proteins, but is not limited thereto.

Further, the peptide-nucleic acid hybrid molecule can be administered by various routes of administration depending on the therapeutic purpose and method of formulation, preferably, by intravenous administration, but it is not limited thereto. Further, the peptide-nucleic acid hybrid molecule can be, but is not limited to, a hybrid ligand, hotspot peptide-incorporating aptamer scaffold, receptor-mimetic hybrid ligand, or hACE2-mimetic hybrid ligand.

In the present invention, in the peptide-nucleic acid hybrid molecule, the nucleic acid is coupled in a click reaction with the hotspot-derived peptide and used to prepare the peptide-nucleic acid hybrid molecule. Further, the nucleic acid can be isolated in vitro from a library of numerous other nucleic acids site-specifically linked to the hotspot peptide by repeated cycles of selection and amplification, wherein nucleic acids capable of providing higher binding to the peptide-nucleic acid hybrid molecule can be selected. Moreover, the nucleic acid can increase the binding affinity of the peptide-nucleic acid hybrid molecule to the binding site (RBD) between the spike protein and the receptor thereof, and for example, can structurally stabilize the hACE2-derived RBD-binding peptide. Furthermore, the nucleic acid may be, but is not limited to, a hexynyl-modified ssDNA, an aptamer scaffold or a hotspot peptide-based aptamer scaffold.

In the present invention, the peptide-nucleic acid hybrid molecule can exhibit excellent binding to RBDs even in competition with RBD binding affinity reagents, and can exhibit excellent binding even when the binding site (RBD) of the target protein is mutated. Specifically, the peptide-nucleic acid hybrid molecule may exhibit strong binding to the RBD of a variant of concern (VOC) of SARS-CoV-2, thereby neutralizing SARS-CoV-2, but is not limited thereto.

In the present invention, the RBD-binding affinity reagents may be, but are not limited to, RBD-binding nucleic acid aptamers, macrocyclic peptides, or monoclonal antibodies. Further, the VOC of SARS-CoV-2 may be, but is not limited to, an alpha (α), beta (β), gamma (γ), delta (δ), or omicron (o) variant.

The present invention provides a peptide-nucleic acid hybrid molecule prepared by the method of the present invention. The peptide-nucleic acid hybrid molecule can block binding of a virus to a host cell receptor, but is not limited thereto.

The present invention also provides a composition for neutralizing viruses, comprising a peptide-nucleic acid hybrid molecule prepared by the method of the present invention.

The present invention also provides a method of inhibiting or neutralizing viruses, comprising the step of administering a peptide-nucleic acid hybrid molecule prepared by the method of the present invention to an individual in need thereof.

### [Advantageous Effects]

According to the method of the present invention, it is possible to rapidly prepare and screen a hotspot-derived peptide-nucleic acid hybrid molecule that can bind with high affinity to viruses and effectively block the binding between the virus and the receptor in response to various virus mutations among numerous candidates. Therefore, it can be used very effectively for the development of therapeutics against various viral mutations.

### [Brief Description of Drawings]

FIG. 1a illustrates the role of the nucleic acid scaffold in inducing stable binding of the hotspot peptide to the virus RBD.
FIG. 1b is a schematic diagram of the in vitro evolution of a receptor mimetic hybrid ligand comprising a hotspot peptide and an aptamer scaffold.
FIG. 2a shows an analysis of the contact residues between the hACE2 receptor and the SARS-CoV-2 RBD.
FIG. 2b depicts the N501Y mutant of RBD, which results in a higher binding affinity for hACE2.
FIG. 2c shows the results of quantitatively assessing RBD binding in the hotspot peptide-bound round 7 (R7) pool: random DNA library binding to hotspot peptides (light gray), R7 ssDNA pool without hotspot peptides (dark gray), and R7 ssDNA pool with hotspot peptides (green).
FIG. 3a shows a comparison of RBD binding fractions for R7-01, R7-02, R7-03 and hybrid randomized library.
FIG. 3b shows the analysis of the RBD binding affinity characterization of R7-02: RBD and serum albumin-coated bead (green and gray, respectively) and bare bead (black).
FIG. 3c shows the results of analysis of the RBD binding affinity properties (K_{d} values) of R7-01 and R7-03.
FIG. 3d shows the energy-minimized structure of R7-02 bound to RBD by molecular docking simulation: LGKGDFR peptide (red), hACE2 (white), RBD (blue), and aptamer scaffold (green).
FIGs. 3e and 3f show the results of analysis of co-incubation and competitive binding of hybrid ligand with RBD-binding nucleic acid aptamers (CoV2-RBD-1C, CoV2-6C3, Aptamer-1), macrocyclic peptide (Peptide 4), or monoclonal antibodies (P05Dhu, AM122).
FIG. 3g shows the results of analysis of the equilibrium dissociation constant K_{d} values of RBD-binding nucleic acid aptamers (CoV2-RBD-1C, CoV2-6C3, Aptamer-1), macrocyclic peptide (Peptide 4), or monoclonal antibodies (P05Dhu, AM122).
FIG. 4a is a schematic diagram and list of mutants of alpha, beta, gamma, delta and omicron variants of concern (VOCs).
FIG. 4b shows the results of analysis of K_{d} values for alpha, beta, gamma, delta and omicron VOCs in R7-02.
FIG. 4c shows the results of analysis of the RBD binding fraction of R7-02 and several types of RBD binders (Aptamer-1, Peptide 4, P05DHu and AM122) against wild-type and omicron VOCs.
FIG. 4d shows the binding fraction of R7-02 and several types of RBD binders (Aptamer-1, Peptide 4, P05DHu and AM122) against wild-type and omicron VOCs via the flow cytometry.
FIG. 5a shows the inhibitory effect of R7-02 on RBD-hACE2 binding by enzyme-linked immunosorbent assay (ELISA).
FIG. 5b shows the inhibition of RBD-hACE2 interaction of LGKGDFR peptide, R7-02 DNA domain, R7-02, random DNA library and hACE2.
FIG. 5c is a schematic illustration of a pseudotype SARS-CoV-2-based neutralization assay.
FIGs. 5d and 5f show confocal microscopy observations of pseudotype SARS-CoV-2 infected hACE2-293T (FIG. 5d: LGKGDFR peptide, R7-02 DNA domain, R7-02; FIG. 5f: CoV2-RBD-1C, CoV2-6C3, Aptamer-1, Peptide 4, P05Dhu, AM122).
FIGs. 5e and 5g show the percentage of GFP-expressing pseudotype SARS-CoV-2 infected cells relative to total hACE2-293T cells (FIG. 5e: LGKGDFR peptide, R7-02 DNA domain, R7-02; FIG. 5g: CoV2-RBD-1C, CoV2-6C3, Aptamer-1, Peptide 4, P05Dhu, AM122).
FIG. 5h shows the results of the nuclease stability analysis of R7-02.

### [Best Mode for Practicing the Invention]

Hereinafter, preferred embodiments of the present invention are provided to facilitate understanding of the present invention. However, the following embodiments are provided only to facilitate understanding of the present invention, but the scope of the present invention is not limited by the following embodiments.

### [Experimental method]

### 1. Materials for experiment

DNA oligonucleotides were synthesized and purified by Bioneer (Korea). The sequences of all oligonucleotides used in the present invention are shown in Table 1 below. C-terminal-(4-azidobutanoyl(lysine))-tagged LGKGDFR peptides (with and without N-terminal FITC modification) were synthesized and purified by Peptron (Korea). EDC (1-ethyl-3-(3-365 dimethylaminopropyl) carbodiimide hydrochloride), NHS (N-hydroxysuccinimide), 1M magnesium chloride solution, human angiotensin-converting enzyme 2 (hACE2), bovine serum albumin (BSA), high glucose DMEM, Tween-20, dimethyl sulfoxide (DMSO), tris(3-hydroxypropyl-triazolylmethyl)amine (THPTA), and copper(II) sulfate pentahydrate were purchased from Sigma-Aldrich. L-Ascorbic acid sodium salt was purchased from Alfa Aesar. Lambda exonuclease enzyme and 10x lambda exonuclease buffer were purchased from Thermo Fisher Scientific. 1M Tris-HCl (pH 7.4) was purchased from Bioneer (Korea). 10x PBS, 8M urea solution, 10x TBE and nuclease-free purified water were purchased from T&I (Korea). 1x PBSMT was used as binding buffer (consisting of 1x PBS having pH of 7.4, containing 2.5 mM MgCl₂ and 0.02% Tween-20 (v/v)). SARS-CoV-2 RBDs of wild type, alpha (B.1.1.7), beta (B.1.351), gamma (P.1) and delta (B.1.617.2) were expressed and purified. The SARS-CoV-2 RBD of omicron (B.1.1.529) was purchased from Abbexa (UK). Alexa-488 labeled monoclonal RBD-conjugated antibody (P05DHu) and Alexa Fluor 488 microscale protein labeling kit were purchased from Invitrogen. Monoclonal RBD-conjugated antibody (AM122) was purchased from ACROBiosystems. The macrocyclic peptide with N-terminal chloroacetyl-modified D-tyrosine (Peptide 4) was linearly synthesized by Peptron (Korea). 1 mg of linear peptide 4 was cyclized by dissolving it in 86 µL of DMSO with 3 µL of N,N-diisopropylethylamine and incubated at 60 °C for 24 hours.

**[Table 1]**

| **ID** | **Sequence (5° → 3')** | **SEQ ID NO.** |
|---|---|---|
| **Forward primer** | [Hexynyl]GGAAGAGATGGCGAC | 1 |
| **Reverse primer** | (Phosphate]CCATCAGGATCAGCT | 2 |
| **Random library** | [Hexynyl]GGAAGAGATGGCGAC-N40-AGCTGATCCTGATGG | 3 |
| **(HTS sequencing) forward primer** | AATGATACGGCGACCACCGAGATCTACACTTGGTGAGACACTCTTTCCCTACA CGACGCTCTTCCGATCTNNNNNNNGGAAGAGATGGCGAC | 4 |
| **(HTS sequencing) reverse primer** | CAAGCAGAAGACGGCATACGAGATGTTCAGGTCTGACTGGAGTTCAGACGTG TGCTCTTCCGATCTNNNNNNCCATCAGGATCAGCT | 5 |
| **R7-01 DNA domain** | [Hexynyl]GGAAGAGATGGCGACCTCCACAACCGCTGATCGACGCAGATCTTG TGGGTAGCTGATCCTGATGG[FAM] | 6 |
| **R7-02 DNA domain** | [Hexynyl]GGAAGAGATGGCGACACGCAATCGTGGGTTGGGGATATAATGGTA AGCTGATCCTGATGG[FAM] | 7 |
| **R7-02 (TAMRA) DNA domain** | [Hexynyl]GGAAGAGATGGCGACACGCAATCGTGGGTTGGGGATATAATGGTA AGCTGATCCTGATGG[TAMRA] | 8 |
| **R7-03 DNA domain** | [Hexynyl]GGAAGAGATGGCGACCACACCAAATCTGAGTCTACCCCATGAAG GAGCTGATCCTGATGG[FAM] | 9 |
| **CoV2-RBD-1C** | [TAMRA]CAGCACCGACCTTGTGCTTTGGGAGTGCTGGTCCAAGGGCGTTAAT GGACA | 10 |
| **CoV2-6C3** | [TAMRA]CGCAGCACCCAAGAACAAGGACTGCTTAGGATTGCGATAGGTTCG G | 11 |
| **Aptamer-1 core** | [TAMRA]TCGAGTGGCTTGTTTGTAATGTAGGGTTCCGGTCGTGGGT | 12 |
| **2'-O-Methyl, 2'-F modified R7-02** | [Hexynyl]G(2'-OMe(G))(2'-OMe-(A))(2'-OMe(A))(2'- OMe(G))AGATGGCGACA CGCAATCGTGGGTTGGGGATATAATGGTAAGCTGATCCT(2'-OMe-(G)) (2'-OMe(A))(2'OMe(T))(2'-F(G))G[FAM] | 13 |
| **Phosphorothioate (*) modified R7-02** | [Hexynyl]G*G*A*A*GAGATGGCGACACGCAATCGTGGGTTGGGGATATAATGG TA AGCTGATCCTG*A*T*G*G[FAM] | 14 |

### 2. Site-specific conjugation of an azide-tagged hotspot peptide and hexynyl-modified ssDNA

For ultra-efficient copper-catalyzed alkyne-azide addition cycloaddition, 1 µL of 50 mM aqueous solution of copper(II) sulfate and 1.67 µL of 150 mM aqueous solution of THPTA were premixed 10 min prior to conjugation. 3 µL of DMSO, 1 µL of 10x PBS, 3 µL of 0.1 mM hexynyl-modified ssDNA aqueous solution, 1 µL of 2.5 mM azide-tagged LGKGDFR peptide, 2.67 µL of premix (copper(II) sulfate and THPTA), and 1 µL of 500 mM sodium ascorbate aqueous solution (freshly prepared) were added in order and mixed thoroughly. The solution was shaken overnight at room temperature and purified by ethanol precipitation.

### 3. Confirmation of the peptide-ssDNA conjugated hybrid library

The formation of peptide-ssDNA conjugation was confirmed by fluorescence after coupling a FITC-modified azide-tagged LGKGDFR peptide. 1 µL of reaction product (~50 ng), 7 µL of 8 M urea water and 1 µL of 6x loading dye were mixed, and heat denatured at 95°C for 10 min. The denatured product was analyzed by electrophoresis in a 10% urea polyacrylamide gel at 300 V for 40 min in 1x TBE buffer and imaged by Azure C600 (Azure Biosystems). An equal amount of unconjugated hexyl-modified ssDNA library was stained with 10,000x SYBR gold staining dye (Invitrogen) and imaged simultaneously.

### 4. HOLD (Hotspot-Oriented Ligand Display)

The peptide-nucleic acid hybrid libraries were thermally denatured at 95 °C for 10 minutes, followed by rapid cooling in ice for the formation of the most stable 3D structures. The thermally treated hybrid libraries were then incubated with RBD-coated beads for 1 hour at room temperature with gentle rotation. To prepare the RBD-coated beads, target RBDs were immobilized on Dynabeads M-270 carboxylic acid (Invitrogen) via an EDC-NHS coupling process using the manufacturer's protocol, and then quantified by a NanoOrange protein quantification kit (Thermo Fisher Scientific). After incubation, magnetic beads were thoroughly washed with 1x PBSMT to remove unbound hybrid molecules, and magnetic bead-bound hybrid ligands were selectively separated with a DynaMag-2 magnet (Invitrogen). Only the DNA domains of the isolated hybrid ligand candidates were PCR amplified using 5'-hexynyl-modified forward primer and 5'-phosphorylated reverse primer (see Table 1 above). The optimal number of PCR cycles was determined by a pilot PCR process. PCR-amplified dsDNA was purified using the QIAquick PCR Purification Kit (Qiagen) and digested with lambda exonuclease to generate a pool of hexynyl-modified ssDNA for the next HOLD round. ssDNA was purified by phenol/chloroform extraction and ethanol precipitation, and quantified by UV-vis measurement at 260 nm. To provide a hybrid library pool of the same format for each round of HOLD, the click reaction between the azide-tagged LGKGDFR peptide and the purified hexyl-modified ssDNA pool was repeated for each round of HOLD.

### 5. Volume dilution challenge

The volume dilution challenge (VDC) technique was used to remove unbound hybrid molecules very efficiently. After 1 hour of incubation and subsequent washing of the bead-bound hybrid library, it was immediately diluted with an increased volume (100-500x) of 1x PBSMT and incubated for an additional 30 minutes to remove hybrid molecules that are easily separated in this process with a high rate dissociation constant (k_{off}) value. After VDC, the beads were collected and washed three times with 200 µL 1x PBSMT.

### 6. Amplification of DNA domains to enrich high-affinity hybrid ligands

The 100 µL PCR reaction mixture consisted of 5 units of tag polymerase (T&I, Korea), 0.5 µL of 0.1 mM 5'-hexynyl-modified forward primer and 5'-phosphorylated reverse primer, 8 µL of 10 mM dNTP (T&I, Korea), 2 µL of 10x PCR buffer (T&I, Korea), 10 µL of collected hybrid ligands, and additional nuclease-free purified water (up to 100 µL). The PCR reaction mixture was pre-denatured at 95 °C for 600 seconds, followed by repeating a cycle of denaturation at 95 °C for 30 seconds, annealing at 51 °C for 30 seconds, and extension at 72 °C for 60 seconds. 5 µL of the PCR mixture was collected and dissolved in a 10% polyacrylamide gel to determine the optimal number of PCR amplification cycles with minimal byproducts (pilot PCR). The DNA domains of the hybrid ligands collected in all rounds of HOLD were PCR amplified with the optimized number of cycles.

### 7. ssDNA generation

After PCR amplification, hexynyl-modified double-stranded DNAs (dsDNAs) were purified using the QIAquick PCR purification kit (Qiagen), and 2.5 µg of purified dsDNA was digested with 5 units of lambda exonucleases (Thermo Fisher Scientific) in 50 µL. The mixture was incubated at 37 °C for 10 min and 80 °C for 10 min, and the hexynyl-modified ssDNA was purified by phenol/chloroform extraction and ethanol precipitation, and quantified by UV-vis measurement at 260 nm.

### 8. Analysis of the relative binding affinity of R7 pools

After 7 rounds of HOLD, the relative binding abilities of the hybrid ligand pools were compared. Equal amounts of peptide-nucleic acid hybrid libraries bound with hotspot peptides, R7 ssDNA pools without hotspot peptides, and R7 ssDNA pools bound with hotspot peptides were heat denatured at 95°C for 10 min, followed by rapid cooling on ice. After thermal denaturation, they were challenged with RBD-coated magnetic beads for 1 hour at room temperature, followed by three washes with 1x PBSMT. RBD binding was analyzed by quantitative PCR (LightCycler 480, Roche). Each PCR reaction contained 10 µL of Light cycler 480 SYBR green I master (Roche), 0.1 µL of 0.1 mM forward and reverse primers, 2 µL of RBD-binding ligand, and 7 µL of nuclease-free purified water. Based on a previously obtained standard curve, the threshold cycle was extrapolated to the amount of RBD-bound ligands.

### 9. High-throughput sequencing

After seven rounds of selection, the enriched pools were PCR amplified with forward and reverse primers containing the adapter sequences, and the optimized number of PCR cycles was determined by pilot PCR. These PCR products were purified using a gel extraction kit (Qiagen) and sequenced by Illumina MiSeq Next Generation Sequencing at SYSGENLAB (Korea). Sequencing libraries were analyzed in 101-bp paired-end (PE) sequencing mode on the Illumina Novaseq6000 platform. Sequencing read quality was measured with the FastQC tool, and adaptor sequences were removed with the cutadapt trimmer with the -e 0.1 -j 20 option. Only perfectly matched-paired reads were used for further analysis. High-quality sequencing reads were passed through the fastq_quality_filter (≥Q30) of the FASTX toolkit (RRID:SCR_005534).

### 10. Analysis of the affinity and specificity of the hybrid ligands

To evaluate the binding affinity of the hybrid ligands to wild-type RBD and its variants, the DNA sequence of R7-02 was synthesized with a 5'-hexynyl group for hotspot peptide conjugation and a 3'-FAM for flow cytometry-based binding assays. FAM-labeled R7-02 in 1x PBSMT was subjected to heat denaturation and rapid cooling. After thermal denaturation, various concentrations of R7-02 were challenged with RBD-coated beads under gentle rotation for 1 hour at room temperature. Beads were washed twice with 1x PBSMT to exclude nonspecific binding, and then bead-bound R7-02 was collected with a DynaMag-2 magnet (Invitrogen). The collected beads were resuspended in 1x PBSMT and analyzed by measuring the average fluorescence of the beads using CytoFLEX S (Beckman Coulter). For each measurement, 15,000 cases were analyzed. Finally, K_{d} was calculated using nonlinear regression analysis (Prism software, GraphPad Prism version 9.3.1). To confirm their binding specificity, similar characterization was performed simultaneously for BSA-coated beads and uncoated beads (bare beads). In the preparation of uncoated beads, Dynabeads M-270 carboxylic acid beads were Tris-blocked in 50 mM Tris-HCl (pH 7.4) for 15 min at room temperature. A similar characterization process was performed for the highly populated sequence families (R7-01 and R7-03) and the reported RBD-binding affinity reagents; Aptamer-1, CoV2-RBD-1C, CoV2-6C3, Peptide 4, P05DHu, and AM122.

### 11. Simulation of the molecular docking of R7-02 in complex with RBD

To analyze the high binding affinity of R7-02 for RBD, molecular docking simulation was used to construct the molecular structure of R7-02 in complex with RBD. The crystal structure of single RBD of hACE2-derived hotspot peptide, LGKGDFR, and SARS-CoV-2 spike protein was obtained from the RCSB PDB database (PDB ID: 6M0J) with a single site mutation (N501Y) in the RBD. The secondary structure of the aptamer scaffold in R7-02 (see Table 1) was predicted using the mfold web server, which was fed into RNAcomposer for 3D structure. The peptide-nucleic acid hybrid structure of R7-02 was constructed by attaching a triazole-modified linker residue between the hexynyl-modified 5' end of the folded DNA and the butanamide-modified lysine C-terminus of the hotspot peptide. Molecular docking simulation was subsequently performed to find the best fit of the hotspot peptide-bound aptamer scaffold to the RBD, while constraining the hotspot peptide and RBD to crystal positions. The resulting structure minimized energy by using the AMBER14 force field utilizing the molecular dynamics simulation package OpenMM version 7.4.

### 12. Competitive binding assay using reported RBD-binding affinity reagents

For competitive binding assays, R7-02 and the reported RBD-binding aptamer were modified with FAM and TAMRA, respectively. Specifically, after thermal denaturation and rapid cooling, they were co-incubated with 5 nM RBD beads (final volume: 100 µL) with gentle rotation for 1 hour at room temperature. Beads were washed three times, and resuspended in 100 µL of 1x PBSMT for analysis with CytoFLEX S (Beckman Coulter). The competitive RBD-binding fraction was calculated as the ratio of the competitive mean fluorescence intensity to the uncompetitive (free-binding) mean fluorescence intensity. For competitive binding with the reported RBD-binding cyclic peptides and antibodies, TAMRA-modified R7-02 was co-incubated with FAM-modified peptide or Alexa-488-modified RBD-antibody and analyzed in the same manner.

### 13. Comparison of binding fractions of wild-type and omicron RBDs

To analyze the binding tolerance of selected RBD binders (Aptamer-1, Peptide 4, P05DHu, and AM122) to SARS-CoV-2 variants, the binding fractions to wild-type and Omicron RBD were quantitatively measured at 10 nM of R7-02 and antibodies (P05DHu and AM122), and at 100 nM of aptamer (Aptamer-1) and cyclic peptide (Peptide 4). RBD-coated magnetic beads were incubated with the respective binders for 1 hour at room temperature with gentle rotation, and then the beads were washed three times and resuspended in 100 µL of 1x PBSMT. The binding fractions of each binder to wild type and Omicron RBDs were measured directly with CytoFLEX S (Beckman Coulter).

### 14. ELISA-based RBD-hACE2 binding inhibition test

To characterize the inhibition efficiency, a SARS-CoV-2 inhibitor screening kit (ACROBiosystems) was used according to the manufacturer's protocol. The microplate was pre-coated with hACE2, and various concentrations of R7-02 (1 pM to 1 µM) were added to the plates. HRP-conjugated SARS-CoV-2 wild-type RBD was added immediately. After 1 hour incubation at room temperature, the well was washed three times and substrate was added to quantify RBD-hACE2 binding. The reaction was terminated by adding stop solution, and absorbance was measured at 450 nm with a microplate reader (Tecan). The value of half the maximum inhibitory concentration (IC₅₀) for R7-02-induced inhibition of RBD-hACE2 binding was determined after log transformation of the hybrid ligand concentration using sigmoidal dose-response nonlinear regression analysis (Prism software, GraphPad Prism version 9.3.1). In the same way, RBD-hACE2 inhibition was compared after the addition of 3 µM of individual ligands (LGKGDFR peptide, DNA domain of R7-02, and R7-02) to analyze the synergistic interaction between the hotspot peptide and the aptamer scaffold in inhibiting RBD-hACE2 interaction.

### 15. Cell culture and sample preparation

The hACE2-293T cell line was obtained from Takara (Japan). The hACE2-293T cells were cultured in high glucose DMEM (Sigma Aldrich) supplemented with 10% fetal bovine serum (Gibco) and 1% sodium pyruvate (Sigma Aldrich) at 37 °C, and 5% CO₂ atmosphere. Subsequently, 5 to 15 passages of hACE2-293T were used in a pseudotype SARS-CoV-2 neutralization assay.

### 16. Neutralization assay for pseudotype SARS-CoV-2

To confirm the neutralization ability of R7-02, a neutralization assay of pseudotype SARS-CoV-2 was performed. hACE2-293T cells (~4X10³) were pre-inoculated into 96-well plates and incubated overnight. After 24 hours, 5 µL of GFP-reporting pseudotype SARS-CoV-2 (4X10⁵ transfection units per mL, BPS bioscience) was pre-incubated with 3 µM of R7-02 in 1x PBSM (1x PBS with 2.5 mM magnesium) for 30 minutes at room temperature. The cell culture medium was removed from the pre-inoculated hACE2-293T cells, and the cells were washed twice with pre-warmed 1x PBSM. After washing, the mixture of pseudovirus and R7-02 was added to the cells and infected for 6 hours at 37 °C. After infection, the 1X PBSM was replaced with fresh cell culture medium, and the cells were incubated at 37°C for another 48 hours. Finally, GFP fluorescence images and bright field images were observed using a confocal microscope (Leica DMi8, source wavelength: 488 nm). The percentage of GFP-expressing transfected cells to total hACE2-293T cells was computationally analyzed by CellProfiler version 4.2.1. The neutralizing efficacy of other RBD-binding ligands (aptamer-1, CoV2-RBD-1C, CoV2-6C3, P05DHu, AM122 and peptide 4) was analyzed by the same method.

### 17. Serum stability assay

To characterize serum stability, nucleases were allowed to degrade FAM-labeled R7-02 in 37 °C, 10% fetal bovine serum for up to 24 hours. For comparison, R7-02 with phosphorothioate backbone introduction, and other modifications such as 2'-O-methyl and 2'-F modifications; and other unmodified single-stranded DNA were tested together. 4 µL of 10 µM sample was mixed with 10x PBS (4 µL), fetal bovine serum (4 µL), and nuclease-free water (28 µL). After 0, 0.5, 2, 4, 8, 18, and 24-hour incubation at 37°C, 5 µL of each aliquot from the different mixtures was immediately treated at 90°C for 5 min and stored in ice before analysis. Aliquots were analyzed by electrophoresis in 10% urea polyacrylamide gel in 1x TBE buffer, stained with 10,000x SYBR gold staining dye (Invitrogen), and imaged by Azure C600 (Azure Biosystems).

### [Embodiment]

### Embodiment 1. Receptor-mimetic hybrid ligand and hotspot-oriented in vitro selection

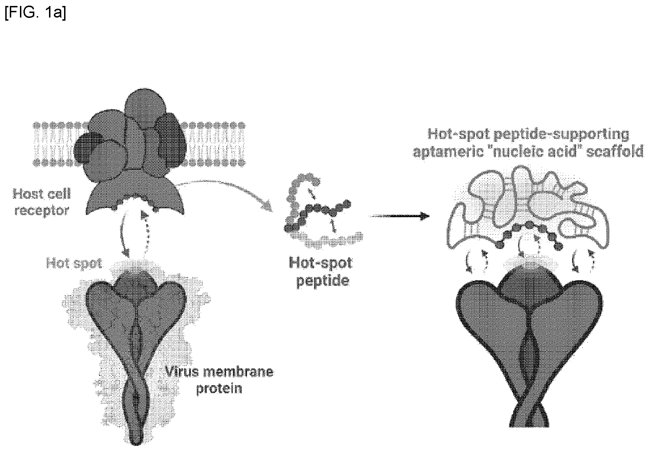

With proper folding, the nucleic acid can provide a highly stable and soluble 3D scaffold that places the hotspot peptide in the correct position and orientation to maintain receptor-like binding properties. This process is shown in FIG. 1a. For the specific RBD-hACE2 interaction, several hotspot peptides have been reported to be approximately 30 amino acids or less in length, but while apart from the insoluble hACE2 transmembrane receptor, flexible motifs cannot bind strongly to the hotspot surface of the receptor-binding domain (RBD). Along with negligible immunogenicity, it is well known that nucleic acids are exceptionally hydrophilic due to the polarity of phosphate backbone. Moreover, systematically designed iterative cycles of selection and amplification can lead to the in vitro selection of unique nucleic acids, capable of structurally stabilizing hACE2-derived RBD-binding peptides. Without large amounts of insoluble domains, a compact and highly available peptide stabilizer would allow high-dose intravenous administration for viral neutralization. Furthermore, the role of nucleic acids can be further extended as hotspot binding enhancers to cooperatively interact with target RBD. That is, there can be additional aptamer-like interactions around the monovalent peptide binding, further enhancing the hotspot binding ability of hACE2-mimetic hybrid ligand.

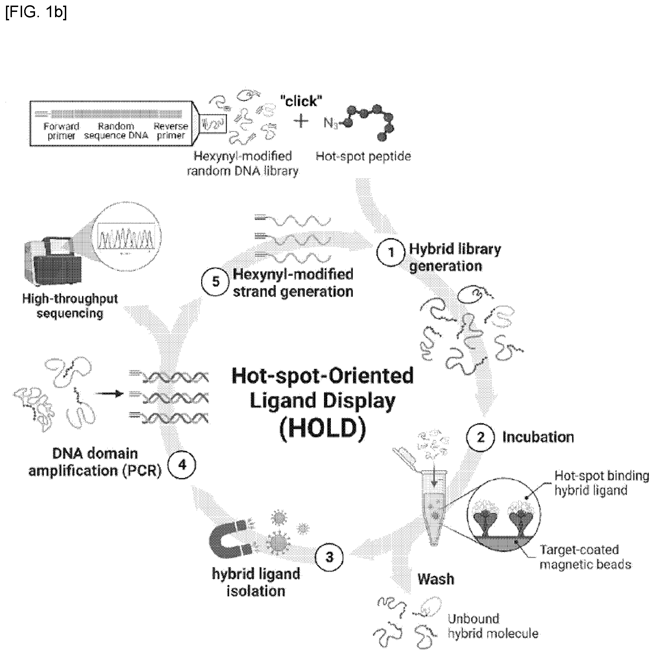

From numerous nucleic acids (~10¹⁴) site-specifically conjugated to the hotspot peptide, the hotspot peptide-supporting aptamer scaffold can be isolated in vitro by repeated cycles of selection and amplification. This process is shown in FIG. 1b.

As shown in FIG. 1b, initially, the azide-tagged C-terminus of the peptide can be site-specifically conjugated to the 5'-hexynyl end of a single-stranded DNA (ssDNA) library by highly efficient copper-catalyzed cycloaddition (step 1 in FIG. 1b). After purification and thermal denaturation, the peptide-nucleic acid hybrid library was rapidly cooled for unique 3D folding of individual ssDNA. Upon introduction of RBD-coated magnetic beads, the RBD surface attracted hotspot interaction of hybrid ligand candidate, and magnetic separation ensured that unbound peptide-DNA conjugate was systematically separated (steps 2-3 in FIG. 1b). Subsequently, the DNA domain of the bead-bound hybrid ligand was selectively amplified by PCR using a 5'-hexynyl-modified forward primer and a 5'-phosphorylated reverse primer (step 4 in FIG. 1b). Using lambda exonuclease, 5'-hexynyl-modified ssDNA was generated, and the click reaction with the azide-tagged hotspot peptide again provided a pool of hybrid libraries in the same format for the next round of the evolutionary HOLD process (step 5 in FIG. 1b).

### Embodiment 2. Preparation of SARS-CoV-2 RBD-binding hotspot peptide, and hACE2 mimic

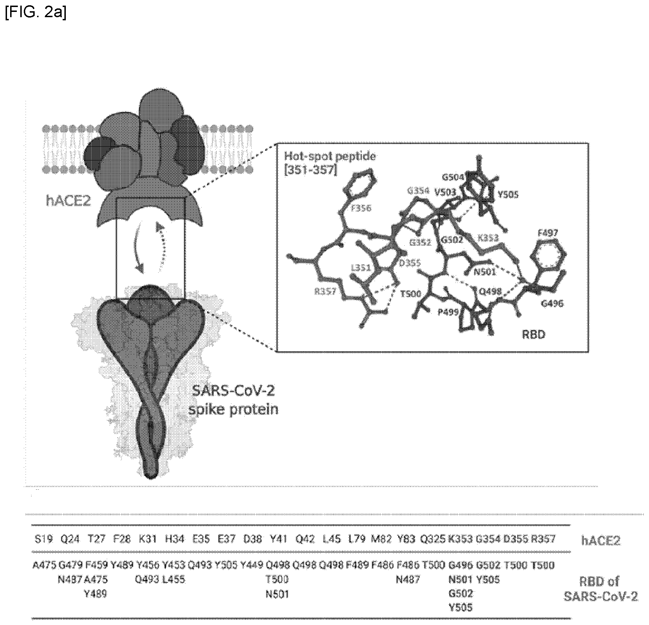

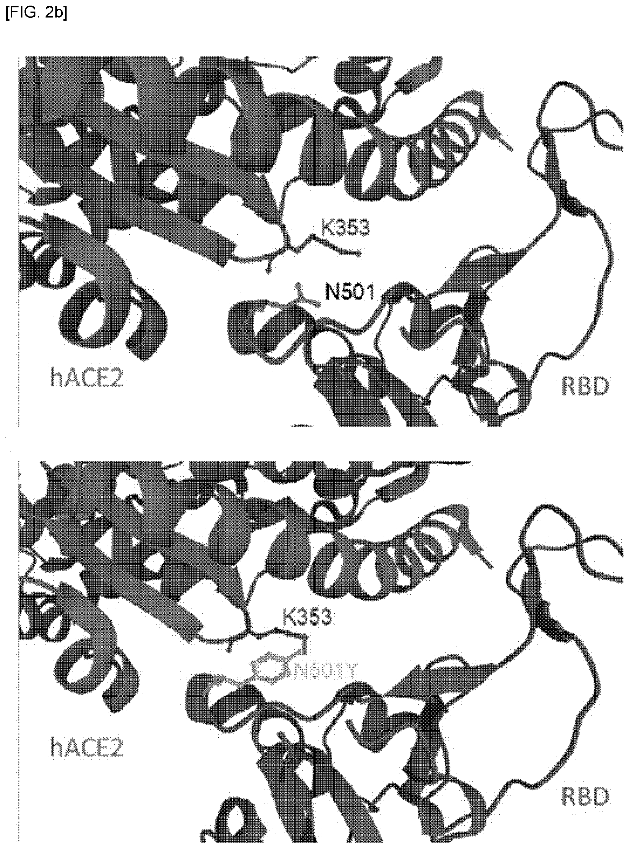

Among the RBD-contacting residues of hACE2, seven amino acid fragments L351 to R357 (LGKGDFR, SEQ ID No. 15) were selected for fusion with a random DNA library due to strong hotspot interaction with the RBD of SARS-CoV-2. Within the short and continuous chain, four amino acids (K353, G354, D355 and R357) were found to be in direct contact with the RBD surface by cryo-electron microscopy (cryo-EM) observation (see FIG. 2a). It is important that the above hotspot region correlates with the binding affinity of SARS-CoV-2 variants of concern (VOCs). Due to the N501Y mutant in the RBD, the VOC binds more tightly and strongly to hACE2, as the central amino acid of the selected hotspot peptide, K353 of hACE2, has an unexpected hydrophobic interaction with the substituted tyrosine (see FIG. 2b). In addition, this LGKGDFR peptide contains two hydrophobic and two positively charged amino acids, which cannot provide the properties of fully hydrophilic and negatively charged DNA. Thus, the hotspot peptide-nucleic acid hybrid library will provide a wide variety of electrostatic interactions as it evolves to have high affinity for the RBD of SARS-CoV-2.

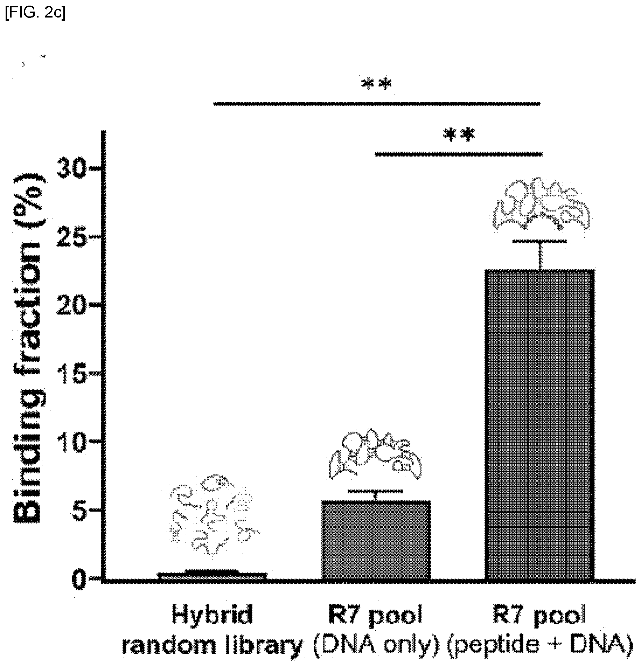

After seven rounds of in vitro selection with progressively increased stringency, the hybrid ligand pool was successfully enhanced to have a strong binding affinity for RBD due to the synergistic effect between the hotspot peptide and the aptamer scaffold. The results are shown in FIG. 2c. Further, the parameters in HOLD of each round are shown in Table 2 below.

**[Table 2]**

| **Round** | **Input hybrid ligand** | **RBD concentration** | **Stringency condition** |
|---|---|---|---|
| **1** | 4ug (200pmol) | 200nM | Washing (200 uL, 3 times) |
| **2** | 2ug (100pmol) | 100nM | Washing (200 uL, 3 times) + VDC (100x dilution, 20 min) |
| **3** | 2ug (100pmol) | 40nM | Washing (200 uL, 3 times) + VDC (500x dilution, 30 min) + Washing (200 uL, 3 times) |
| **4** | 1ug (50pmol) | 40nM | Washing (200 uL, 3 times) + VDC (500x dilution, 30 min) + Washing (200 uL, 3 times) |
| **5** | 1ug (50pmol) | 40nM | Washing (200 uL, 3 times) + VDC (500x dilution, 30 min) + Washing (200 uL, 3 times) |
| **6** | 1ug (50pmol) | 40nM | Washing (200 uL, 3 times) + VDC (500x dilution, 30 min, 2 times) + Washing (200 uL, 3 times) |
| **7** | 1ug (50pmol) | 20nM | Washing (200 uL, 3 times) + VDC (500x dilution, 30 min, 2 times) + Washing (200 uL, 3 times) |

As shown in FIG. 2c, the RBD binding of the hotspot peptide-linked round 7 (R7) pool was quantitatively assessed in a relative binding assay. Despite the linkage of hotspot peptide, the random DNA library exhibited a significantly lower level of RBD binding. When the peptide-nucleic acid hybrid library was challenged with RBD-coated magnetic beads, the binding fraction was only 0.38%, indicating that the hotspot peptide could not bind strongly to RBD without a stable scaffold. In the absence of the LGKGDFR peptide, the R7 pool slightly increased RBD binding, resulting in a binding fraction of 5.74%, presumably due to its own aptamer binding effect. On the other hand, the peptide-based R7 pool dramatically enhanced its affinity for RBD, and its binding fraction (22.6%) was 59.37-fold and 3.94-fold higher than the random DNA-peptide conjugate and peptide-deficient R7 pool, respectively. This significant improvement in RBD binding is attributed to the synergistic interaction between the hotspot peptide and the newly evolved aptamer scaffold.

### Embodiment 3. Identification and characterization of peptide-supporting aptamer scaffold

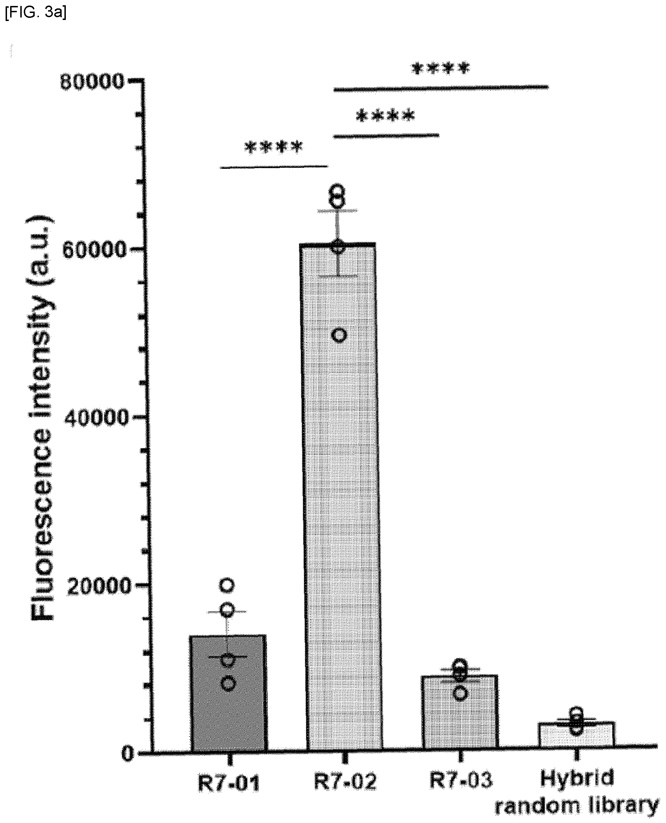

The most optimal peptide-supporting aptamer scaffold was identified in the R7 hybrid ligand pool using high-throughput sequencing and flow cytometry-based binding assay. When more than 4 million DNAs were sequenced in the R7 pool, the three most abundant sequence families were found to account for a high percentage (>18%). These are shown in Table 3 below (SEQ ID Nos. 16 to 22). Among the three sequence families, the most representative DNA sequences were synthesized with a 5'-hexynyl group for site-specific peptide conjugation and a 3'-FAM dye for flow cytometry-based binding assay. For the 5'-linked hotspot peptide, the second most abundant DNA sequence (the DNA domain of R7-02) was the strongest in RBD binding. In the relative binding analysis, the RBD binding fraction of R7-02 was approximately 5-fold and 8-fold greater than those of R7-01 and R7-03, respectively (see FIG. 3a).

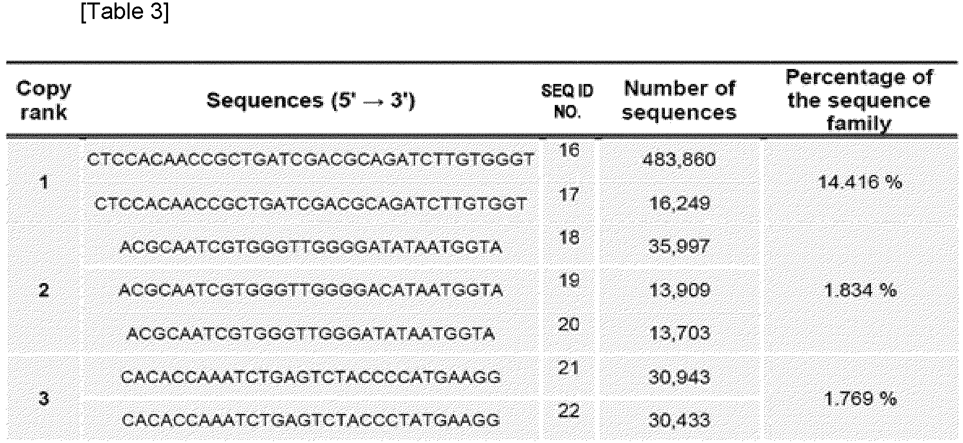

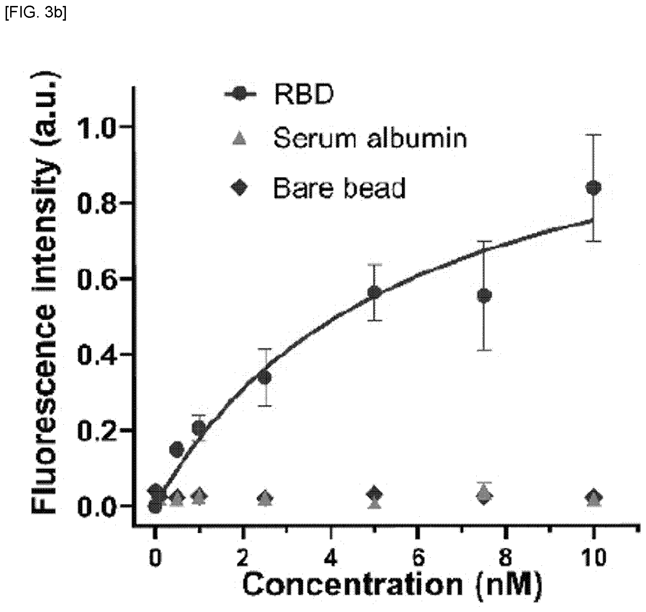

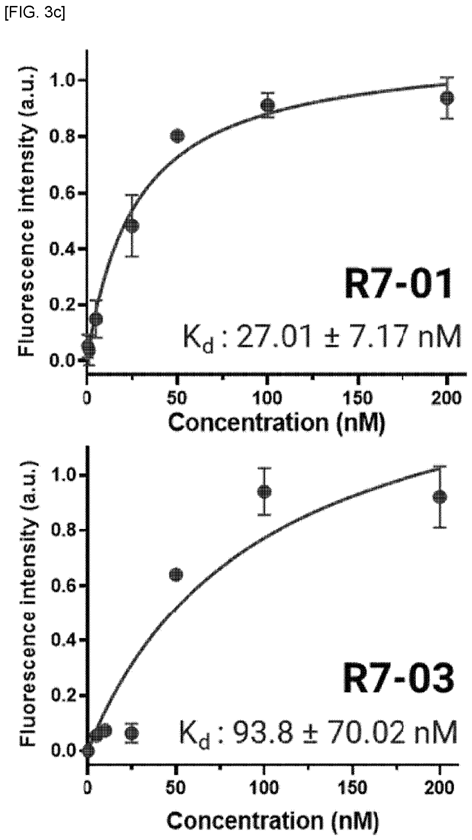

To act as a hACE2 mimetic, the most strong hybrid ligand, R7-02, showed high affinity and specificity for RBD at the correct site for hACE2 to bind. When the K_{d} of R7-02 was quantitatively measured, it was 5.702 nM for RBD (see FIG. 3b, green), while the K_{d} values of R7-01 (27.01 nM) and R7-03 (93.8 nM) were much higher than R7-02 (see FIG. 3c).

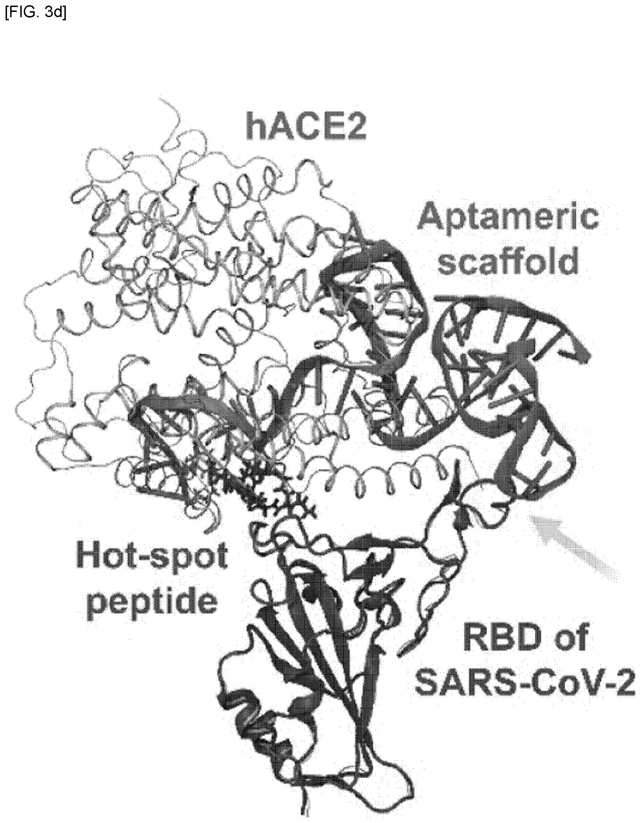

In addition, molecular docking simulation was performed and the result is shown in FIG. 3d.

As shown in Fig. 3d, it was predicted that the high binding affinity of R7-02 for RBD would be attributed to the cooperative binding effect. In the energy minimization simulation snapshot, the LGKGDFR peptide (red) was precisely located at the binding hotspot of the original hACE2 (white) and RBD (blue), and the binding was effectively stabilized by the conjugated aptamer scaffold (green). DNA folding at the 3' end provided an additional binding motif in the loop structure (A475-N487) (yellow arrow) of RBD, leading to strong and specific RBD binding of R7-02. The precise positioning of R7-02 at the interface between RBD and hACE2 demonstrated its potential for neutralizing SARS-CoV-2 at low nanomolar concentrations in physiological body fluids.

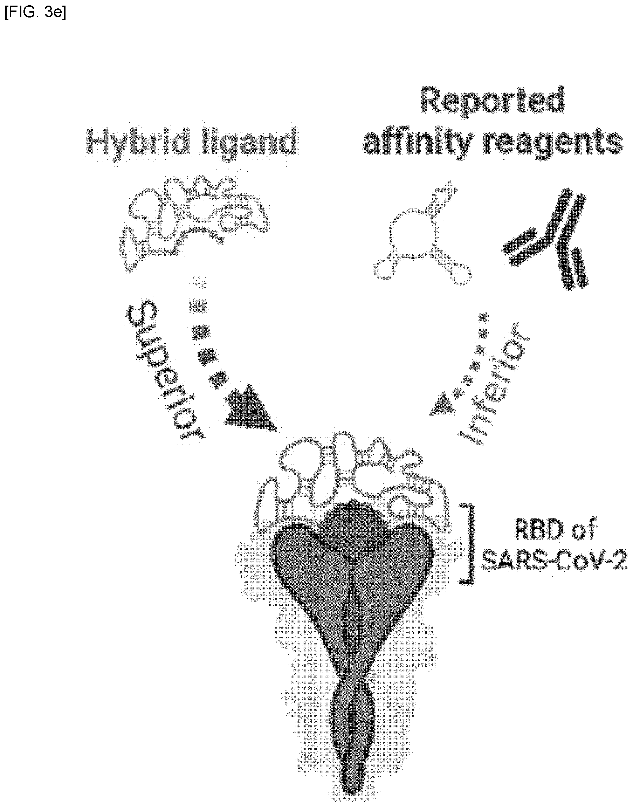

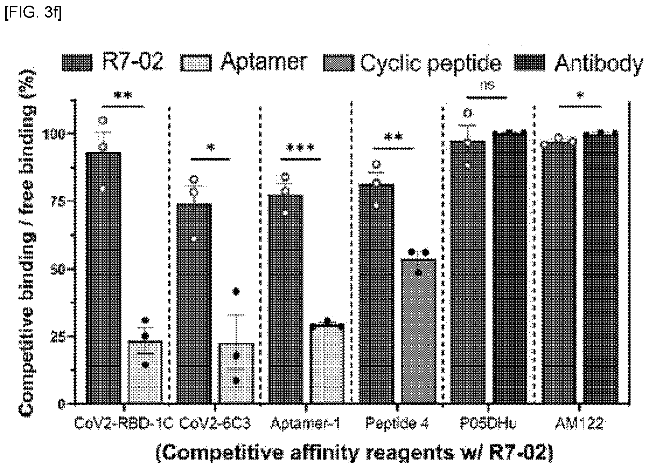

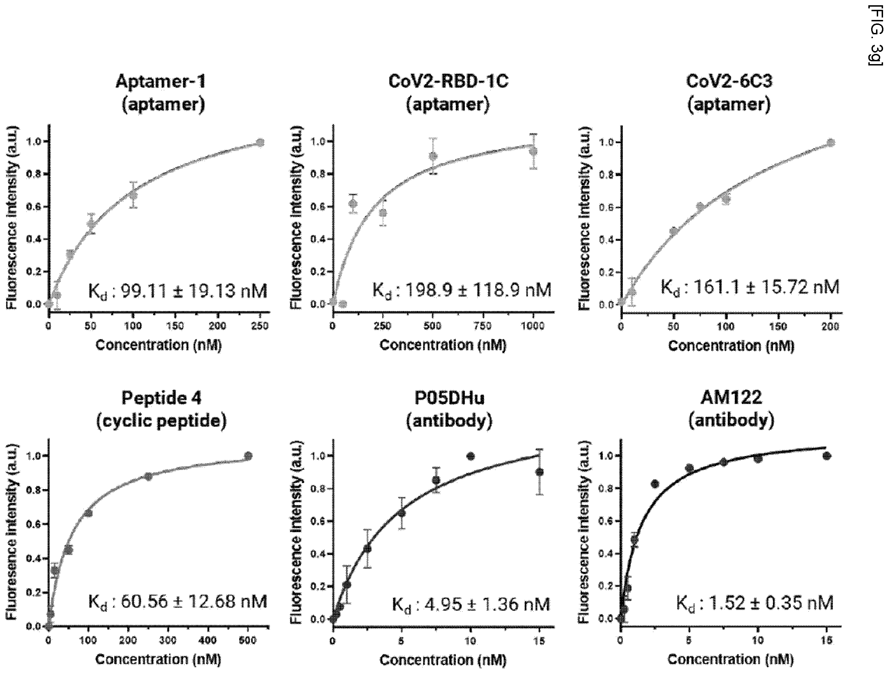

Even in competition with previously reported RBD-binding affinity reagents, R7-02 exhibited sustainable retained binding on the target surface of RBD (see FIGs. 3e and 3f). To induce RBD binding competition, hybrid ligand of hotspot peptide and aptamer scaffold was co-incubated with RBD-binding nucleic acid aptamers (CoV2-RBD-1C, CoV2-6C3, Aptamer-1), macrocyclic peptides (Peptide 4), or monoclonal antibodies (P05DHu and AM122). Most of them are known to block the binding interface between RBD and hACE2 (see FIG. 3e). Also, the K_{d} values of various RBD binders under the same conditions for R7-02 were confirmed (see FIG. 3g).

To assess competitive binding, the fraction of RBD-bound R7-02 with or without potential binding competitors was measured, and the reduction in RBD-binding was quantitatively compared to the reduction in other affinity reagents (see FIG. 3f). After 1 hour of co-incubation with RBD-binding aptamers or cyclic peptides, R7-02 was able to maintain RBD binding, and the ratio of competitive binding to free binding was reduced to a low level (approximately 6.8%). Meanwhile, the RBD binding of all other aptamers and cyclic peptides was significantly inhibited, with the ratio between competitive and free binding decreasing by up to 77.2%, indicating that the binding of monomeric aptamers and cyclic peptides is relatively weak to occupy restrictive RBD binding sites for competition with cooperative hybrid ligands. Furthermore, due to the different binding sites, the competition between R7-02 and P05DHu and AM122 antibodies was insignificant.

### Embodiment 4. Binding tolerance of hACE2 mimics to SARS-CoV-2 variants of concern (VOCs)

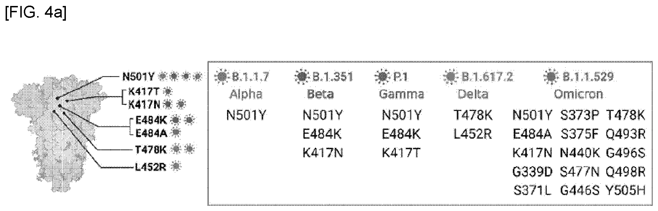

The hotspot peptide-nucleic acid hybrid molecule (R7-02) was found to bind strongly to all reported VOCs of SARS-CoV-2 due to its high variant tolerance to binding. Although initially evolved to bind to wild-type RBDs via the HOLD process, the binding ability of the hybrid ligand is also capable of recognizing highly mutated RBDs of VOCs. This allows them to interact strongly with the hotspot interface of the RBD. VOC was found to have several key mutants in RBD, including N501Y, which is highly interactive with the lysine of the LGKGDFR peptide in the hybrid ligand, enhancing its binding to the hACE2 receptor and transmissibility into the host cell (see FIG. 4a).

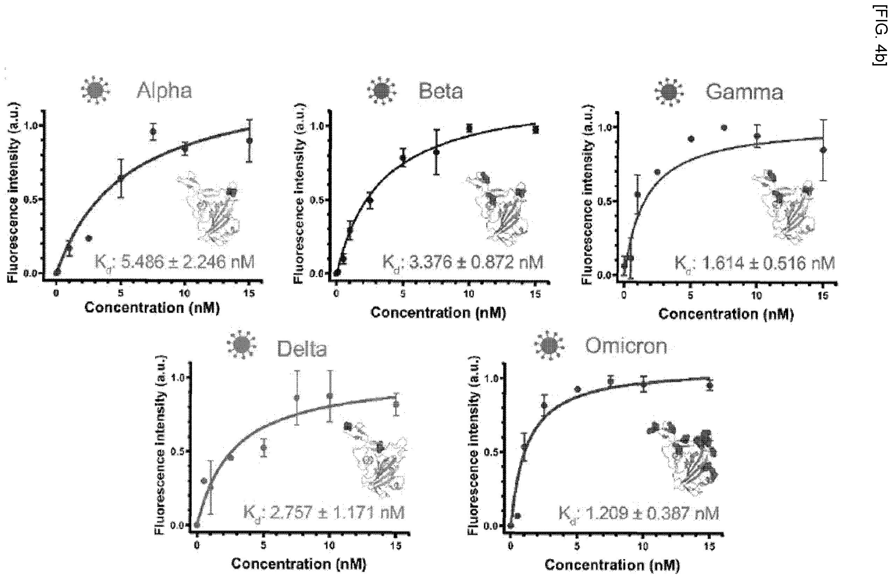

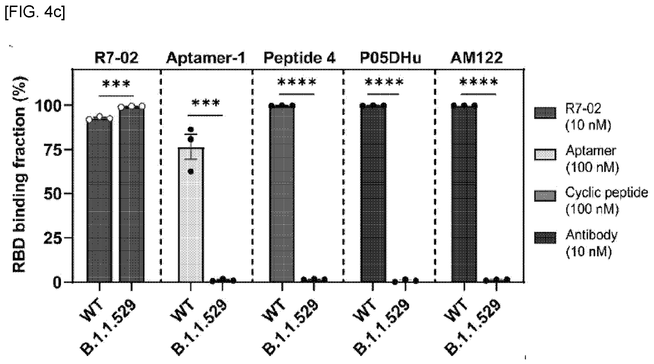

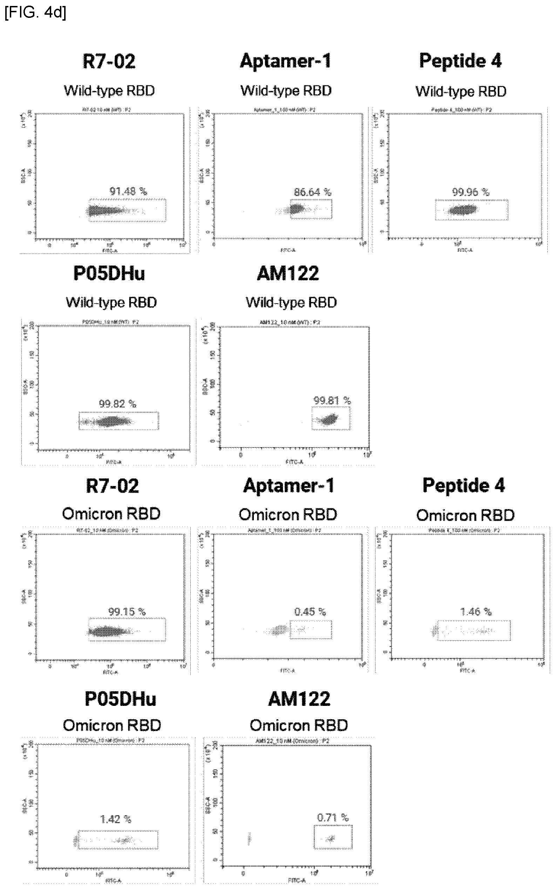

When the binding of R7-02 to the five VOCs was quantitatively assessed, the K_{d} values of R7-02 (alpha, beta, gamma, delta, and omicron; 5.486 nM, 3.376 nM, 1.614 nM, 2.757 nM, and 1.209 nM) were lower than those of wild-type RBD (5.702 nM), confirming the binding tolerance of R7-02 to all variants (see FIG. 4b). Interestingly, Omicron with the most RBD mutations was most strongly recognized by R7-02, while several selected types of RBD binders (Aptamer-1, Peptide 4, P05DHu, and AM122) lost their ability to bind RBD by severe mutations (see FIGs. 4c and 4d) in variants.

Accordingly, the binding tolerance of R7-02 to SARS-CoV-2 VOCs was confirmed. Further, it indicates that hotspot peptide-derived RBD binding may be affected by an enhanced hACE2 recognition mechanism by SARS-CoV-2 VOCs.

### Embodiment 5. Inhibition of RBD-hACE2 interaction and neutralization of pseudotype SARS-CoV-2

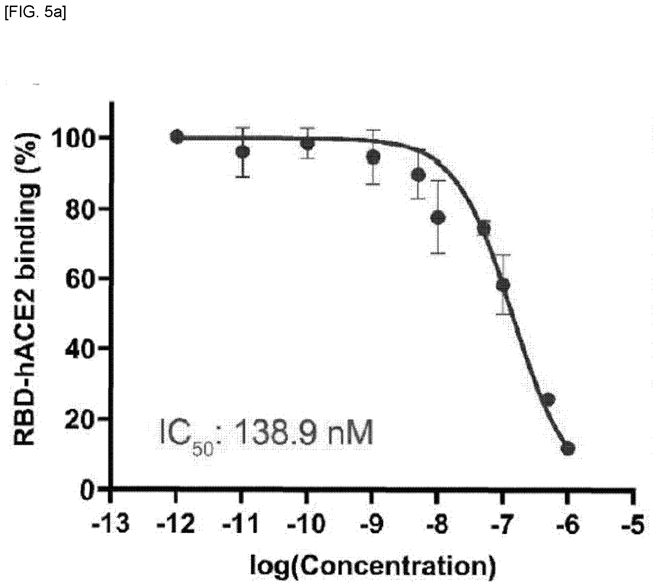

Due to the strong binding to RBD, the hotspot peptide-nucleic acid hybrid molecule efficiently inhibited the interaction between RBD of SARS-CoV-2 and hACE2 receptor even at nanomolar concentrations. To characterize the inhibition efficiency, an enzyme-linked immunosorbent assay (ELISA) was performed while varying the concentration of R7-02 of 1 pM to 1 µM, and R7-02 exhibited a half-maximal inhibitory concentration (IC₅₀) of 138.9 nM for inhibition of RBD-hACE2 binding (see FIG. 5a).

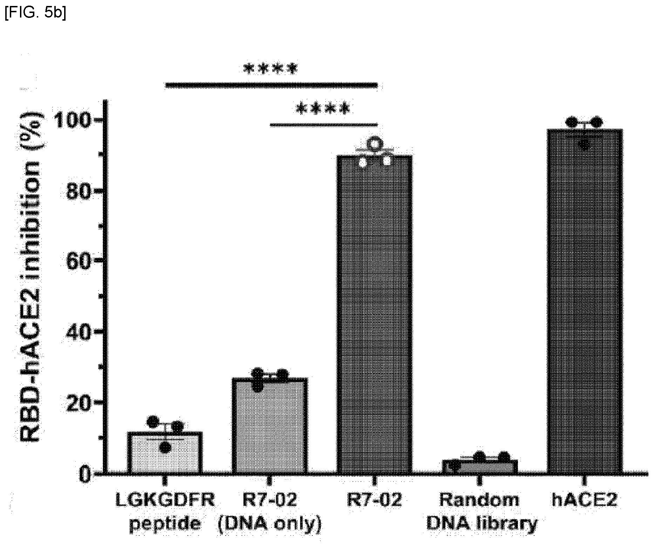

Meanwhile, the RBD inhibition of R7-02 was guided by the synergistic effect of the systematically combined hotspot peptide and the in vitro evolved aptamer scaffold; the individual components of R7-02, the LGKGDFR peptide and the aptamer scaffold, showed 11.69% and 26.82% inhibition of RBD-hACE2 interaction at 3 µM concentration, whereas the whole structure of R7-02 showed 89.60% inhibition. As an evidence of the aptameric behavior, the DNA scaffold alone showed better inhibition efficiency compared to the random DNA library (inhibition rate of 3.77%) (see FIG. 5b).

Previously, several RBD-binding affinity reagents were unable to block the precise hACE2 contact residues on the RBD surface, thus failing to effectively inhibit RBD-hACE2 interactions. In contrast, the hotspot-derived RBD binding of R7-02 demonstrated efficient inhibition of RBD in hACE2 binding, indicating great potential for SARS-CoV-2 neutralization.

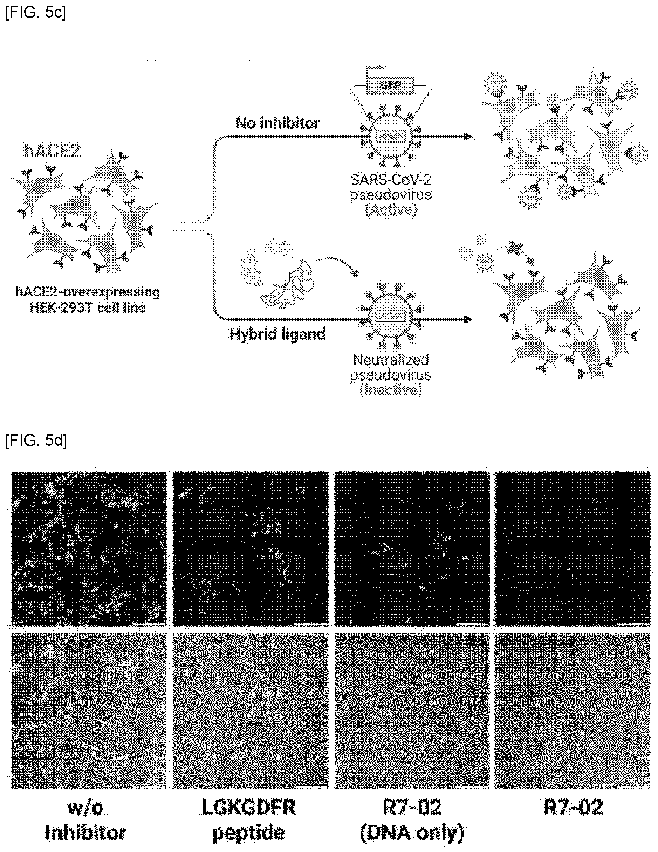

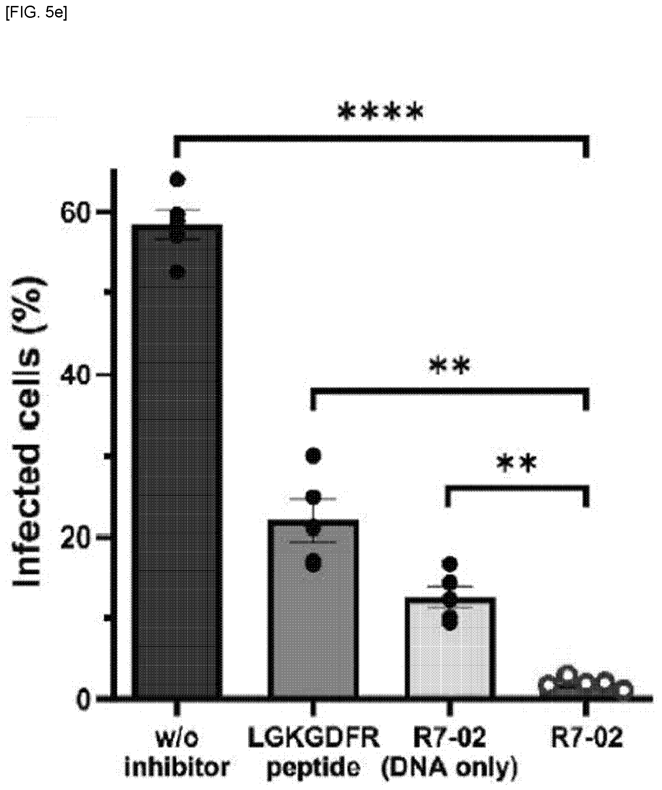

Therefore, the SARS-CoV-2 neutralization ability of the hotspot peptide-nucleic acid hybrid molecule was further confirmed. For the neutralization assay, a pseudotype SARS-CoV-2 that expresses both the SARS-CoV-2 spike protein and green fluorescent protein (GFP) but lacks the ability to replicate itself was prepared. The neutralization efficiency of R7-02 was analyzed fluorescently by measuring the fluorescence intensity of GFP, which is only expressed in cells infected by pseudotype SARS-CoV-2 (see FIG. 5c). Briefly, the hACE2-overexpressing HEK-293T cell line (hACE2-293T) was pre-inoculated into microplates overnight and then challenged with pseudotype SARS-CoV-2 (0.5 multiplicity of infection, MOI) for 6 hours for viral infection. The results are shown in FIGs. 5d and 5e.

As shown in FIGs. 5d and 5e, in the absence of RBD inhibitors, hACE2-293T was infected with pseudotype SARS-CoV-2, with quantitatively 58.4% of all cells emitting strong green fluorescence as an evidence of successful viral infection. In contrast, in the presence of either the aptamer scaffold or hotspot peptide consisting of R7-02, the level of RBD binding was limited, reducing the infection rate with pseudotype SARS-CoV-2 to 22.0% and 12.6%, respectively. In addition, R7-02, a hotspot peptide-based aptamer scaffold, exhibited negligible green fluorescence and a significantly lower percentage of infected cells (1.9%).

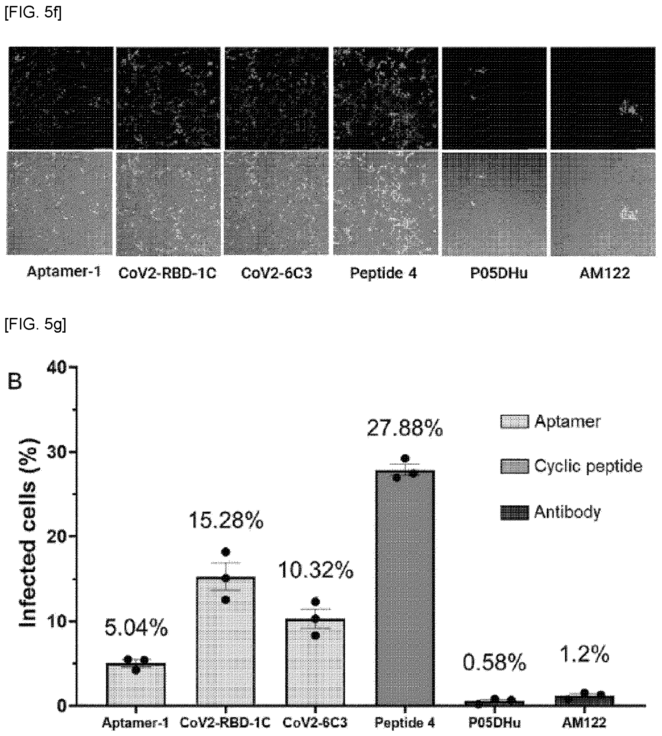

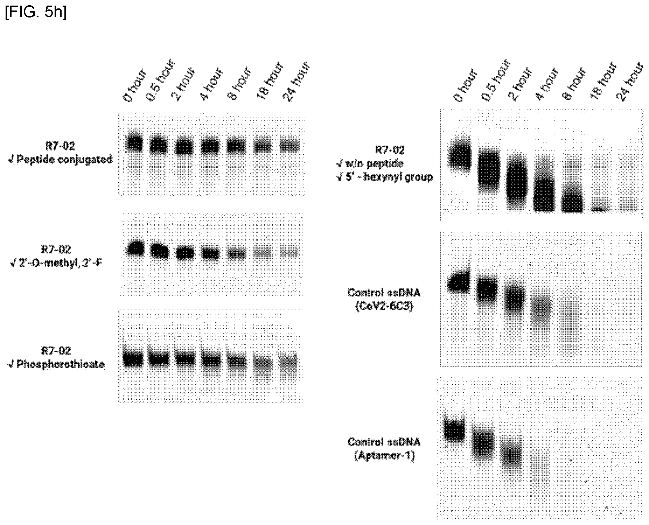

When compared to the viral neutralization of many different RBD binders, the neutralizing efficiency of R7-02 were found to be significantly higher or comparable (see FIGs. 5f and 5g). On the other hand, it should be considered that, unlike the most commonly used neutralizing antibodies, the hybrid ligand of the present invention is fully synthetic. That is, the hybrid ligand of the present invention has a low molecular weight (20 kDa), which is one-tenth of that of monoclonal antibodies, but it ensures efficient infection inhibition. In addition, without further modification, the chimeric structure of the peptide-aptamer hybrid ligand showed good nuclease resistance and serum stability compared to well-known oligonucleotide modifications (e.g., phosphorothioate backbone, 2'-O-methyl and 2'-F modifications) (see FIG. 5h), indicating great potential as SARS-CoV-2 neutralizer.

The foregoing description of the present invention is for illustrative purposes only, and one having ordinary skill in art can understand that it can be readily adapted to other specific forms without changing the technical idea or essential features of the present invention. Therefore, it should be understood that the embodiments described above are in all respects exemplary and not limiting.

## Claims

1. A method of preparing a target protein-binding peptide-nucleic acid hybrid molecule, comprising the following steps:
(a) site-specifically conjugating a hotspot-derived peptide with a random nucleic acid library to prepare a peptide-nucleic acid hybrid;
(b) co-incubating the target protein-coated magnetic beads with the peptide-nucleic acid hybrid of step (a); and
(c) screening peptide-nucleic acid hybrid molecules that bind to target proteins using magnets.

2. The method according to claim 1, wherein the method further comprises the following steps:
(d) selectively amplifying and cloning the nucleic acid portion of the peptide-nucleic acid hybrid molecule selected in step (c) above;
(e) preparing the double-stranded DNA generated in step (d) above to a single-stranded DNA by using a exonuclease, and purifying it; and
(f) preparing a new randomized nucleic acid library consisting of the single-stranded DNA of step (e) above, and repeating the selection, amplification, and purification processes of steps (a) to (f) above.

3. The method according to claim 1, wherein, after step (a), the method further comprises the step of thermally denaturing and cooling the peptide-nucleic acid hybrid to induce 3D folding of the nucleic acid.

4. The method according to claim 1, wherein the hotspot-derived peptide of step (a) above is a peptide having at least one functional group selected from the group consisting of azido lysine, azidobutanoic acid, azinoacetic acid, and azide at the C-terminus or N-terminus,
wherein the randomized nucleic acid library of step (a) above comprises a single-stranded nucleic acid having at least one functional group selected from the group consisting of hexynyl, 5-octadiynyl, and alkyne at the 5' end,
wherein the hotspot-derived peptide and the single-stranded nucleic acid are site-specifically conjugated by a click reaction.

5. The method according to claim 1, wherein the hotspot-derived peptide of step (a) above is a peptide having at least one functional group selected from the group consisting of hexynyl, 5-octadiynyl, and alkyne at the C-terminus or N-terminus,
wherein the randomized nucleic acid library of step (a) above comprises a single-stranded nucleic acid having at least one functional group selected from the group consisting of azido lysine, azidobutanoic acid, azinoacetic acid, and azide at the 5' end,
wherein the hotspot-derived peptide and the single-stranded nucleic acid are site-specifically conjugated by a click reaction.

6. The method according to claim 2, wherein the amplification of the nucleic acid portion of step (d) above utilizes a forward primer with one or more functional groups selected from the group consisting of hexynyl, 5-octadiynyl and alkyne.

7. The method according to claim 1, wherein the random nucleic acid library has a following structure:
5'-functional group-forward primer-[Nₓ]-reverse primer-3',
wherein the functional group is selected from the group consisting of hexynyl, 5-octadiynyl, alkyne, azido lysine, azidobutanoic acid, azinoacetic acid, and azide,
wherein N is A, T, C or G, and x is an integer of 25 to 100.

8. The method according to claim 1, wherein the hotspot-derived peptide is an amino acid of a binding site between a receptor and a target protein thereof.

9. The method according to claim 1, wherein the target protein is a viral envelope protein, a growth factor protein, a cell membrane receptor, an antibody, or an antigenic protein.

10. The method according to claim 9, wherein the viral envelope protein is selected from the group consisting of a spike protein, a fiber protein, and an envelope glycoprotein.

11. The method according to claim 9, wherein the virus is selected from the group consisting of Coronavirus, influenza virus, Hepatitis virus, Human Immunodeficiency virus (HIV), Human Papillomavirus, Herpesvirus, and Ebolavirus, MERS virus, Rotavirus, Hantavirus, Monkeypox virus, Adenovirus, Rabies virus, and Norovirus.

12. The method according to claim 11, wherein the coronavirus is selected from the group consisting of human coronavirus 229E (HCoV-229E), human coronavirus OC43 (HCoV-OC43), severe acute respiratory syndrome coronavirus (SARS-CoV), human coronavirus NL63 (HCoV-NL63, New Haven coronavirus), human coronavirus HKU1, Middle East respiratory syndrome coronavirus (MERS-CoV), severe acute respiratory syndrome coronavirus 2 (SARS-CoV-2), and variants thereof.

13. The method according to claim 1, wherein the peptide-nucleic acid hybrid molecule binds to a binding site between a receptor and a target protein thereof.

14. The method according to claim 1, wherein the peptide-nucleic acid hybrid molecule satisfies any one of the following features:
(a) nuclease resistance; and
(b) serum stability.

15. The method according to claim 1, wherein the peptide-nucleic acid hybrid molecule neutralizes viruses.

16. The method according to claim 1, wherein the nucleic acid enhances binding affinity to a binding site between a receptor of the peptide-nucleic acid hybrid molecule and a target protein thereof.

17. A peptide-nucleic acid hybrid molecule prepared by the method of any one of claims 1 to 16.

18. The peptide-nucleic acid hybrid molecule according to claim 17, wherein the peptide-nucleic acid hybrid molecule is for blocking binding of a virus to a host cell receptor.

19. A composition for neutralizing viruses comprising a peptide-nucleic acid hybrid molecule prepared by the method of any one of claims 1 to 16.

20. A method of inhibiting or neutralizing viruses, comprising the step of administering a peptide-nucleic acid hybrid molecule prepared by the method of any one of claims 1 to 16 to an individual in need thereof.
